(19) Europäisches Patentamt
European Patent Office
Office européen des brevets

(11) **EP 4 064 288 A1**

(12) **EUROPEAN PATENT APPLICATION**

(43) Date of publication:
**28.09.2022 Bulletin 2022/39**

(21) Application number: **21170607.2**

(22) Date of filing: **27.04.2021**

(51) International Patent Classification (IPC):
**G16H 20/10** (2018.01)     **G16H 50/20** (2018.01)
**G01N 33/574** (2006.01)     **G16C 20/30** (2019.01)

(52) Cooperative Patent Classification (CPC):
**G16H 20/10; G01N 33/5011; G16C 20/30;
G16H 50/20;** G01N 2800/52; G16C 20/70

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.03.2021 KR 20210037449**

(71) Applicant: **MBD Korea. Co., Ltd.
Gyeonggi-do 16229 (KR)**

(72) Inventors:
• **KU, Bo Sung
16939 Yongin-si (KR)**
• **KIM, Jungeun
16813 Yongin-si (KR)**

(74) Representative: **Goddar, Heinz J.
Boehmert & Boehmert
Anwaltspartnerschaft mbB
Pettenkoferstrasse 22
80336 München (DE)**

(54) **SYSTEM FOR EVALUATING ANTICANCER DRUG SENSITIVITY AND COMPUTER-READABLE MEDIUM STORING PROGRAM EXECUTING EVALUATION METHOD**

(57)     The system for evaluating sensitivity to an anticancer drug according to the embodiment of the present invention includes a communication unit configured to receive biological test data of a biological specimen isolated from a biological individual and a processor connected to the communication unit, where the processor is configured to determine anticancer drug sensitivity of the biological individual in terms of whether treatment response is positive or negative based on the biological test data, by using a sensitivity prediction model configured to determine sensitivity to an anticancer drug based on an anticancer drug response factor and a cell growth factor, and then to provide results of evaluation on the sensitivity of the biological individual to the anticancer drug.

FIG. 2d

(5)

**Description**

**TECHNICAL FIELD**

The present invention relates to a system for evaluation of anticancer drug sensitivity and a computer-readable medium storing programs executing an evaluation method for anticancer drug sensitivity, and more particularly to a system and a recording medium configured to evaluate anticancer drug sensitivity based on multiple prediction factors associated with anticancer drug sensitivity.

**BACKGROUND ART**

Treatment for diagnosed cancer may generally include surgery, chemotherapy, and radiation therapy. Chemotherapy treatment based on anticancer drugs, *inter alia,* is a great option for cancer treatment, but resistance of cancer cells to the anticancer drugs is emerging as a new problem with the chemotherapy.

More specifically, resistance to anticancer drugs can occur through different mechanisms such as reducing accumulation of the drug in the cell, activating detoxification or efflux of the drug, or altering the drug target protein by cells exposed to the anticancer drugs for a long time. This process can be not only a large obstacle to curative cancer therapy, but also very deeply related to the failure of the cancer treatment.

Particularly, when chemotherapy is attempted in cancer patients and any anticancer agent does not work, the cancer patient may have a resistance to other anticancer drugs as well. Further, combination chemotherapy, which is using a combination of two or more different drugs each having a different mechanism at a time, may not be effective even if it is tried in the initial cancer treatment.

In other words, evaluation on the sensitivity of a biological individual to an anticancer drug can be essential in determining the direction of the treatment for cancer patients. It is therefore continuously required to develop a system for evaluation of sensitivity to anticancer drugs in a biological individual.

The background art of the invention is given for the better understanding of the present invention. It should not be construed to consider that the matters specified in the background art of the present invention exist as the prior art.

**DISCLOSURE OF INVENTION**

**TECHNICAL PROBLEM**

On the other hand, an evaluation system based on $IC_{50}$ to evaluate the sensitivity to an anticancer drug has been newly proposed as a way to evaluate sensitivity to the anticancer drug, in which the value of $IC_{50}$ is defined as the concentration of the anticancer drug where the viability of a cell is reduced by half (50 %) based on the 100 % viability of the cell which does not have treatment using the drug.

More specifically, the system for evaluation of anticancer drug sensitivity based on $IC_{50}$ may be configured to predict the treatment response of a biological individual by treating a cancer cell derived from the biological individual with an anticancer drug and then determining the value of $IC_{50}$ which is the concentration of the drug where the growth of the cancer cell is reduced by 50 %.

But, the conventional system for evaluation of anticancer drug sensitivity based on the value of $IC_{50}$ may have a considerably low accuracy of prediction as it considers only the value of $IC_{50}$ associated with the efficacy of the anticancer drug other than the growth rate of the target cell.

Especially, when comparing the therapeutic efficacy of an anticancer drug between the cancer cells of different biological individuals, the system for evaluation of anticancer drug sensitivity based on the value of $IC_{50}$ does not consider the cell growth factor (for example, cell growth rate) of the biological individuals, so the accuracy of prediction for sensitivity may deteriorate.

For this reason, the inventors of the present invention give attention to the fact that there are limitations to the prediction of the treatment response to an anticancer drug in clinical trials when the sensitivity to the anticancer drug is evaluated with the value of anticancer drug response, $IC_{50}$ alone.

In connection to this, the inventors of the present invention have recognized that it is possible to overcome the limitations of the conventional system for evaluation of anticancer drug sensitivity based on the value of $IC_{50}$ alone by considering a cell growth factor such as cell growth rate in addition to a drug response factor as factors in predicting the treatment response to an anticancer drug and thereby reflecting the growth rate of the cell in the prediction.

As a result, the inventors of the present invention have developed a system for evaluation of anticancer drug sensitivity based on multiple prediction factors.

More specifically, the inventors of the present invention were able to design a system for evaluation of anticancer drug sensitivity to evaluate the sensitivity to an anticancer drug based on multiple factors such as a drug response factor and a cancer cell growth factor. It is therefore expected to provide evaluation results with such a high level of reliability that they match the clinical trial results in the biological individual.

In this regard, the inventors of the present invention have further applied a sensitivity prediction model to a novel system for evaluation of anticancer drug sensitivity, where the sensitivity prediction model is designed to receive biological test data of a biological specimen derived from a biological individual as input data, determine an anticancer drug, drug response factors (e.g., $IC_{50}$, $\%IC_{50}$, or the rate of change of cell viability) and cell growth factors (e.g., cell growth rate or Ki67) and output the degree of sensitivity to the anticancer drug based on the drug response factors and the cell growth factors.

More specifically, the degree of treatment response can be determined based on "cell growth factors" consisting of the cell growth rate and the growth-related marker such as ki67 and "anticancer drug response factors" consisting of $IC_{50}$, $\%IC_{50}$ (= ($IC_{50}$/highest concentration) x 100), cell viability at a certain drug concentration A (= (cell viability at concentration A / cell viability without drug treatment) x 100), and the rate of change of cell viability at a certain drug concentration A (= (cell viability / initial cell viability) x 100).

More specifically, the cell viability can be determined as a quantitative measurement by using general cell viability dyes (Calcein AM), cell immunochemical dyes (cell viability-related marker such as F-action), colorimetric cell viability assays (MTS, MTT, or CCK-8), fluorescent emission (luminescent such as cell titer glo), and so forth.

Therefore, the inventors of the present invention have contrived a novel system for evaluation of anticancer drug sensitivity to predict the sensitivity to an anticancer drug, so it can be expected to evaluate the sensitivity of a biological individual to an anticancer drug with high accuracy.

The inventors of the present invention have particularly contrived the evaluation system configured to calculate the sensitivity index to a specific anticancer drug based on the shortest distance to a prediction dividing line (or fitting line), which is defined as a boundary line for prediction of the treatment response, so it can evaluate the treatment response to the anticancer drug in a biological individual (specimen) existing in the boundary of the prediction dividing line.

The treatment response to an anticancer drug may be assessed depending on the sensitivity index to the anticancer drug as follows:

Sensitivity index -100% ~ -10%: low treatment response,
Sensitivity index -10% ~ 10%: middle treatment response, and
Sensitivity index 10% ~ 100%: high treatment response

In other words, the present invention is contrived to cope with the above-specified technical problems and has an object to provide a method for evaluation of sensitivity to an anticancer drug and a system using the evaluation method that is designed to evaluate the anticancer drug sensitivity of a biological individual with high accuracy and thus capable of compensating for the different problems caused by the limitations and drawbacks of the prior art.

**TECHNICAL SOLUTION**

In order to achieve the object of the present invention, there is provided a system for evaluating anticancer drug sensitivity in accordance with one embodiment of the present invention. The evaluation system includes a communication unit configured to receive biological test data according to cell experiments of a biological specimen isolated from a biological individual, and a processor connected to the communication unit. In this regard, the processor is configured to determine sensitivity to an anticancer drug in the biological individual in terms of whether treatment response is positive or negative based on the biological test data by using a sensitivity prediction model configured to determine sensitivity to an anticancer drug based on an anticancer drug response factor and a cell growth factor according to cell experiments, and then to provide evaluation results on the sensitivity to the anticancer drug in the biological individual.

In accordance with a feature of the present invention, the biological test data according to cell experiments may be at least one selected from the group consisting of the name of the anticancer drug, the concentration of the anticancer drug, the dilution ratio of the anticancer drug, and cell viability.

In accordance with another feature of the present invention, the sensitivity prediction model may be further configured to extract a sensitivity characteristic associated with the anticancer drug response factor and the cell growth factor based on the biological test data according to cell experiments; and the processor may be further configured to use the sensitivity prediction model to extract the sensitivity characteristic based on the biological test data and determine the anticancer drug sensitivity of the biological individual in terms of whether treatment response is positive or negative.

In accordance with further another feature of the present invention, the evaluation system may further include a receiver for receiving reference data of clinical trial results for the biological individual, and the processor may be further configured to use the sensitivity prediction model to determine the anticancer drug sensitivity of the biological individual in terms of whether treatment response is positive or negative based on the biological test data and the reference data.

In accordance with further another feature of the present invention, the processor may be further configured to use the sensitivity prediction model to calculate the degree of treatment response to the anticancer drug in terms of an anticancer drug sensitivity index, determine prediction results for the anticancer drug sensitivity as being at least one of high, middle and low based on the degree of treatment response to the anticancer drug, and provide the degree of treatment response to the anticancer drug determined by the sensitivity prediction model.

In accordance with further another feature of the present invention, the processor may be further configured to authenticate a user intending to receive the results of evaluation on the anticancer drug sensitivity of the biological individual.

In accordance with further another feature of the present invention, the sensitivity prediction model may be based on at least one algorithm selected from the group consisting of LR (Logistic regression), PR (Probit regression), Quadratic classifiers, Kernel estimation, LVQ (Learning vector quantization), ANN (Artificial neural networks), RF (random forest), Bagging (bootstrap aggregating), AdaBoost, Gradient Boosting, XGBoost, SVM (support vector machine), LASSO (least absolute shrinkage and selection operator), Ridge (ridge regression), and Elastic Net.

In accordance with further another feature of the present invention, the anticancer drug may be a drug used for anticancer therapy, including doxorubicin, and the biological individual may be an entity with cancer, such as ovarian cancer, lung cancer, stomach cancer, or breast cancer.

In order to achieve the object of the present invention as described above, there is also provided a computer-readable medium storing a program executing a method for evaluation of anticancer drug sensitivity according to one embodiment of the present invention. The evaluation method, which is a method of evaluating the anticancer drug sensitivity as implemented by a processor, includes: (a) receiving biological test data of a biological specimen isolated from a biological individual; (b) determining anticancer drug sensitivity of the biological individual in terms of whether treatment response is positive or negative based on the biological test data by using a sensitivity prediction model configured to determine anticancer drug sensitivity based on an anticancer drug response factor and a cell growth factor; and (c) providing results of evaluation on the anticancer drug sensitivity of the biological individual.

In accordance with a feature of the present invention, the biological test data according to cell experiments may be at least one selected from the group consisting of the name of the anticancer drug, the concentration of the anticancer drug, the dilution ratio of the anticancer drug, and cell viability.

In accordance with another feature of the present invention, the sensitivity prediction model may be further configured to extract a sensitivity characteristic associated with the anticancer drug response factor and the cell growth factor based on the biological test data according to cell experiments. The step of determining the anticancer drug sensitivity in terms of whether treatment response is positive or negative may further include: using the sensitivity prediction model to extract the sensitivity characteristic based on the biological test data; and determining the anticancer drug sensitivity of the biological individual in terms of whether treatment response is positive or negative based on the sensitivity characteristic.

In accordance with further another feature of the present invention, the sensitivity characteristic may be a fitting line created by performing a curve fitting for the biological test data including the cell growth factor and the anticancer drug response factor of the cell. In this case, the term " cell growth factor" is a concept that includes all the factors representing cell proliferation, such as cell growth rate; and the term "anticancer drug response factor" is a concept that includes all the factors representing the efficacy of the anticancer drug, such as $IC_{50}$, $\%IC_{50}$, cell viability rate, or the rate of change of cell viability.

In accordance with further another feature of the present invention, the evaluation method may further include additionally receiving reference data of clinical trial results for the biological individual prior to determining the anticancer drug sensitivity in terms of whether treatment response is positive or negative. In addition, the step of determining the anticancer drug sensitivity in terms of whether treatment response is positive or negative may further include using the sensitivity prediction model to determine the anticancer drug sensitivity of the biological individual in terms of whether treatment response is positive or negative based on the biological test data and the reference data.

In accordance with further another feature of the present invention, the step of determining the anticancer drug sensitivity in terms of whether treatment response is positive or negative may further include using the sensitivity prediction model to determine the degree of treatment response to the anticancer drug as being at least one of high, middle and low. Also, the step of providing the results of evaluation on the anticancer drug sensitivity may include providing the degree of treatment response to the anticancer drug determined by the sensitivity prediction model.

In accordance with further another feature of the present invention, the evaluation method may further include authenticating a user intending to receive the results of evaluation on the anticancer drug sensitivity of the biological individual, prior to receiving the biological test data.

In accordance with further another feature of the present invention, the sensitivity prediction model may be based on at least one algorithm selected from the group consisting of LR (Logistic regression), PR (Probit regression), Quadratic classifiers, Kernel estimation, LVQ (Learning vector quantization), ANN (Artificial neural networks), RF (random forest), Bagging (bootstrap aggregating), AdaBoost, Gradient Boosting, XGBoost, SVM (support vector machine), LASSO (least absolute shrinkage and selection operator), Ridge (ridge regression), and Elastic Net.

In accordance with further another feature of the present invention, the anticancer drug may be a drug used for cancer therapy, including doxorubicin, and the biological individual may be an entity with cancer, such as ovarian cancer, lung cancer, stomach cancer, or breast cancer.

The specific features of other embodiments are included in the detailed description and the accompanying drawings.

## EFFECTS OF INVETNION

The present invention provides a system for evaluation of anticancer drug sensitivity that is configured to evaluate the treatment response of a biological individual to an anticancer drug with high accuracy and output the results of evaluation and overcomes the limitations of the conventional system for evaluation of anticancer drug sensitivity based on the value of $IC_{50}$.

More specifically, the present invention can overcome the limitations of the conventional evaluation system for anticancer drug sensitivity based on $IC_{50}$ that has a very considerably low accuracy of prediction as a result of taking into account the efficacy of the anticancer drug simply by using $IC_{50}$ but not considering the degree of growth of the target cell.

In particular, the present invention can provide analysis results with high accuracy by providing a novel system for evaluation of anticancer drug sensitivity using a sensitivity prediction model configured to receive biological test data of a biological specimen isolated from a biological individual as input data, determine the type of a desired anticancer, $IC_{50}$, and cell growth factors and output the degree of anticancer drug sensitivity based on the results of the determination.

Furthermore, the present invention offers a system for evaluation of anticancer drug sensitivity based on multiple factors such as anticancer drug response factors and growth factors of cancer cells and hence provides evaluation results with such a high level of reliability that they match the clinical trial results for the biological individual.

In other words, the present invention can provide analysis results with high reliability in the evaluation on the anticancer drug sensitivity, which is fundamental to determine the direction of treatment for cancer patients. Medical workers are therefore allowed to choose a suitable anticancer drug for conditions of a biological individual with ease, and the present invention is expected to contribute to excellent prognosis.

The effects of the present invention are not limited to the illustrative description and more various effects are included in the present invention.

## BRIEF DESCRIPTION OF DRAWINGS;

FIG. 1a is an exemplary illustration of a system for evaluation of treatment response to an anticancer drug based on a system for evaluation of anticancer drug sensitivity according to an embodiment of the present invention.

FIG. 1b is an exemplary illustration showing the configuration of the system for evaluation of anticancer drug sensitivity according to an embodiment of the present invention.

FIG. 1c is an illustration showing the configuration of a user system receiving information about the results of an evaluation on the anticancer drug sensitivity from the system for evaluation of anticancer drug sensitivity according to an embodiment of the present invention.

FIGS. 2a to 2f are exemplary illustrations showing the procedures of a method for evaluation of anticancer drug sensitivity according to an embodiment of the present invention.

FIG. 3 is an exemplary illustration showing the evaluation results of the system for evaluation of anticancer drug sensitivity according to different embodiments of the present invention.

## BEST MODES FOR CARRYING OUT THE INVENTION

The following content is to merely illustrate the principles of the invention. It is therefore possible for the skilled in the art to invent a variety of devices that implement the principles of the invention and are included in the conception and scope of the invention, although not definitely specified or illustrated in this specification. All the conditional terminologies and embodiments as mentioned in this specification are intended only for better understanding of the conception of the invention and not construed to limit the particularly specified embodiments and conditions.

In the following description, the ordinal numbers "first", "second", or so forth are used to describe equivalent and independent entities and not construed to have any meaning of main/sub or master/slave in the order.

The objects, advantages and features of the present invention will become more apparent from the following detailed description of the invention taken in conjunction with the accompanying drawings, and it is therefore possible for those skilled in the art to implement the technical conception of the present invention with ease.

The individual features of the different embodiments of the present invention can be partially or entirely coupled or combined with each other and, as fully understood by the skilled in the art, susceptible to various ways of technical connection and driving. The individual embodiments are to be implemented alone or in conjunction.

For clear interpretation of this specification, the terminologies used in the description of the present invention will be defined as follows.

The term "biological individual" as used herein may refer to any entity subjected to evaluation in regards to anticancer drug sensitivity. For example, the biological individual may be an entity with at least one cancer selected from the group consisting of ovarian cancer, breast cancer, squamous cell cancer, uterine cancer, cervical cancer, prostate cancer, head and neck cancer, pancreatic cancer, brain tumor, liver cancer, skin cancer, esophageal cancer, testicular cancer, kidney cancer, colon cancer, rectal cancer, stomach cancer, kidney cancer, bladder cancer, bile duct cancer, and gallbladder cancer. Preferably, the biological individual may be, if not limited to, an entity subjected to an evaluation on the sensitivity to an anticancer drug of doxorubicin or an entity with ovarian cancer or breast cancer. Furthermore, the biological individual disclosed herein may be, if not limited to, any mammals other than human.

The term "anticancer drug" as used herein refers to a chemotherapeutic agent used to inhibit proliferation of cancer cells, and it may include chemical anticancer drugs or targeted anticancer drugs. For example, the anticancer drug may be, as well as doxorubicin, paclitaxel, taxotere, adriamycin, endostatin, angiostatin, mitomycin, bleomycin, cisplatin, carboplatin, daunorubicin, idarubicin, 5-fluorouracil, methotrexate, actinomycin-D, or a combination thereof. Preferably, the anticancer drug as used herein may be, if not limited to, doxorubicin.

The term "anticancer drug sensitivity" as used herein may mean sensitivity to an anticancer drug or a measure of evaluation about whether a targeted anticancer drug causes a treatment response. In this specification, the anticancer drug sensitivity is interchangeable with treatment response or drug response. According to the results of evaluation on the anticancer drug sensitivity, the biological individual may be evaluated as having a positive treatment response or a negative treatment response to a specific anticancer drug. More specifically, a biological individual with a relatively high sensitivity to an anticancer drug is evaluated as having a positive treatment response; whereas a biological individual with a relatively low sensitivity to an anticancer drug is evaluated as having a negative treatment response.

In this regard, the anticancer drug sensitivity may have something to do with the growth rate of the cancer cell isolated from a biological individual in addition to the drug response of the cancer cell, i.e., $IC_{50}$. More specifically, the degree of drug response (the degree of drug efficacy) of the cancer cell may be inversely proportional to the growth rate of the cancer cell. It is to say that the accuracy and sensitivity of the evaluation on the anticancer drug sensitivity are likely to be high when taking into consideration the drug response factor and the growth rate of a cancer cell according to cell experiments that are quantitative indexes of drug efficacy, rather than a single factor. In particular, the results of an anticancer sensitivity evaluation based on multiple factors such as anticancer response factors and cancer cell growth factors are provided with such a high level of reliability that they match the clinical trial results for the biological individual.

The term "anticancer drug response factor" as used herein is the measure that shows the quantitative measurement of the cell-based response to an anticancer drug, and includes cell viability according to the anticancer.

For example, the cell viability is determined by staining living cells and measuring the total intensity/size and the selected intensity/size of a fluorescent substance, or by staining dead cells and measuring apoptosis for inverse calculation of cell viability.

The cell viability can also be determined by measuring the transformation of cells or a solution containing the cells using a chemical factor, such as MTT or APT.

The term "cell growth factor" as used herein includes the quantitative index that shows the growth of cells over time. For example, the cell growth factor can be determined by measuring the cell viability over time.

In this regard, the growth factor may be the rate of increase in cell viability, the cell growth rate, the rate of increase in cell size, or the colony generation rate. The term "cell growth rate" as used herein may mean a change in the volume, size or number of cells over a defined period of time.

In this regard, the cell growth rate may be calculated only for specific cells. And, the cell growth factor may be calculated only for a cell group in which cell division or growth is active among a number of cells.

The term "biological test data" as used herein may mean test data according to cell experiments or test conditions of a biological specimen isolated from a biological individual. In this regard, the biological specimen may be at least one selected from the group consisting of tissue, cell, whole blood, serum, plasma, saliva, cerebrospinal fluid, and urine.

Preferably, the biological test data in this specification may be test data and/or test condition data of a cancer cell (or cancer tissue) isolated from a biological individual. For example, the biological test data may include at least one raw data associated with cell viability after treatment with an anticancer drug as selected from the name, concentration and dilution ratio of the anticancer drug, cell viability, and further anticancer treatment condition data. But the biological test data are not limited to the disclosure above.

In accordance with the feature of the present invention, when the biological specimen is a cell, the cell may be cultured on a culture plate that includes a space for containing a cell culture solution, a plurality of pillar portions projecting in the form of pillars with a defined height from the flat bottom surface and designed to have a cell as a culture subject placed on, and a trench groove portion being concavely formed on the flat bottom surface in the other way of the outwardly bulging pillar portions so that the cell culture solution placed in the containing space is kept from lying around all over the bottom but accumulates at one end of the bottom. The cell cultured on the culture plate is then subjected to a biological test for evaluation in regards to anticancer drug sensitivity, thereby acquiring biological test data after a biological test. In connection with the cell culture, Public Patent Notification No. 10-2020-0055230 is attached herein as a reference. The culture plate and the culture conditions for cell culture are not limited to the disclosure of this invention.

The term "sensitivity prediction model" as used herein may refer to a model configured to receive anticancer response factors and cell growth factors or biological test data according to cell experiments as input data and output the degree of sensitivity to an anticancer drug.

More specifically, the sensitivity prediction model may be a model instructed to receive biological test data associated with the anticancer response factors and the cell growth factors according to cell experiments as input data, extract a sensitivity characteristic from the biological test data, and predict the degree of sensitivity to an anticancer drug based on the extracted sensitivity characteristic.

In accordance with a feature of the present invention, the sensitivity prediction model may be configured to receive reference data of clinical trial results as input data and predict the degree of anticancer drug sensitivity based on the sensitivity characteristic extracted from the biological test data according to cell experiments and the reference data of clinical trial results.

In this regard, the reference data of clinical trial results may include, but are not limited to, reference data of $IC_{50}$, growth factor and clinical trial results.

In accordance with another feature of the present invention, the sensitivity prediction model may consist of an input module, an analysis module, and an output module.

More specifically, the input module may be configured to receive biological test data and furthermore reference data of clinical trial results as input data. The analysis module may be configured to extract sensitivity characteristic from the biological test data and evaluate the degree of anticancer drug sensitivity based on the sensitivity characteristic and/or reference data of clinical trial results. Further, the output module may be configured to output the evaluated degree of anticancer drug sensitivity.

In accordance with further another feature of the present invention, the curve fitting may be performed based on at least one of LR (Logistic regression), PR (Probit regression), Quadratic classifiers, Kernel estimation, LVQ (Learning vector quantization), ANN (Artificial neural networks), RF (random forest), Bagging (bootstrap aggregating), AdaBoost, Gradient Boosting, XGBoost, SVM (support vector machine), LASSO (least absolute shrinkage and selection operator), Ridge (ridge regression), and Elastic Net. Preferably, the sensitivity prediction model may be, but not limited to, a classification model based on LR algorithm.

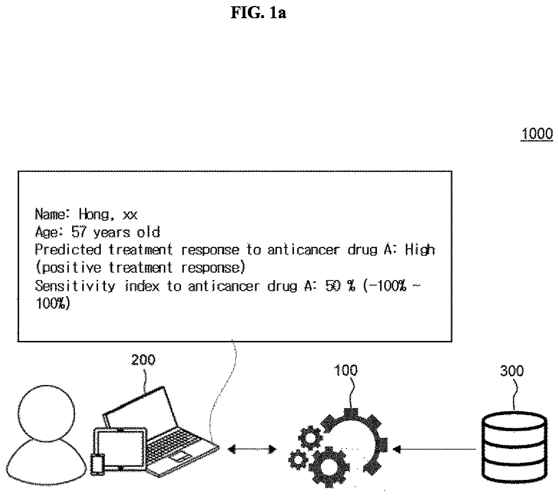

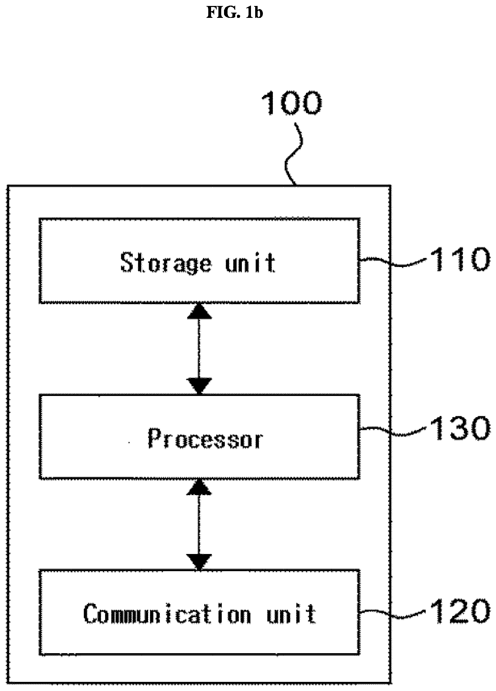

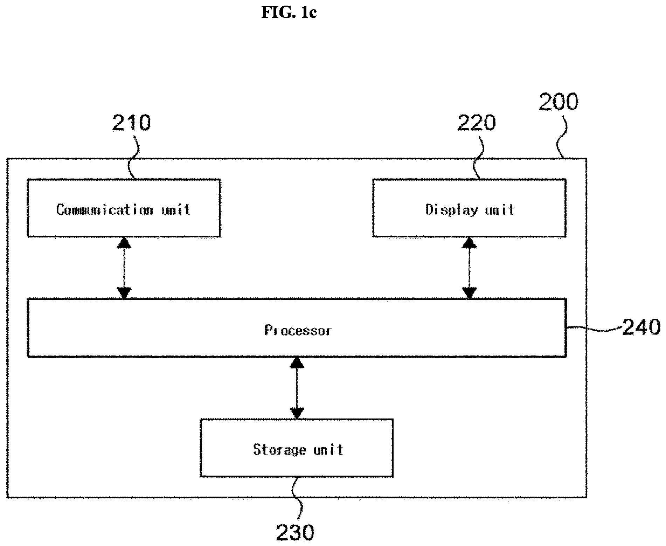

Hereinafter, a detailed description will be given as to a system for evaluation of treatment response to an anticancer drug based on an evaluation system for anticancer drug sensitivity according to an embodiment of the present invention with reference to FIGS. 1a, 1b and 1c.

FIG. 1a is an exemplary illustration of a system for evaluation of treatment response to an anticancer drug based on an evaluation system for anticancer drug sensitivity according to an embodiment of the present invention. FIG. 1b is an exemplary illustration showing the configuration of the evaluation system for anticancer drug sensitivity according to an embodiment of the present invention. FIG. 1c is an exemplary illustration showing the configuration of a user system configured to receive and output information about the results of an evaluation on the anticancer drug sensitivity from the system for evaluation of anticancer drug sensitivity according to an embodiment of the present invention.

Referring to FIG. 1a, a system 1000 for evaluation of treatment response to an anticancer drug may be a system configured to provide information about the sensitivity to an anticancer drug based on the biological test data of a biological individual. In this regard, the system 1000 for evaluation of treatment response to an anticancer drug may be comprised of a system 100 for evaluation of anticancer drug sensitivity configured to determine the degree of sensitivity of a biological individual to an anticancer drug based on biological test data; a user system 200 for receiving information about the sensitivity to the anticancer drug; and a database providing server 300 for providing the biological test data and/or reference data of clinical trial results.

Most of all, the system 100 for evaluation of anticancer drug sensitivity may include a general-purpose computer, a laptop, and/or a data server for executing various arithmetic operations in order to determine the degree of sensitivity to the anticancer drug based on the user's biological test data and/or the reference data of clinical trial results received from the database providing server 300. The user system 200 may be, if not limited to, a system for access to a web server for providing a web page regarding the evaluation of anticancer drug sensitivity or a mobile web server for providing a mobile web site.

More specifically, the system 100 for evaluation of anticancer drug sensitivity may receive biological test data from the database providing server 300 and provide information associated with the degree of sensitivity to the anticancer drug based on the received biological test data. In this regard, the system 100 for evaluation of anticancer drug sensitivity may be configured to perform an evaluation of anticancer drug sensitivity based on a sensitivity prediction model. For example, the system 100 for evaluation of anticancer drug sensitivity may be configured to evaluate a sensitivity index to anticancer drug A in addition to a predicted treatment response to anticancer drug A.

The system 100 for evaluation of anticancer drug sensitivity may send information about the evaluation of the anticancer drug sensitivity of a biological individual to the user system 200.

The data received from the system 100 for evaluation of anticancer drug sensitivity may be provided in the form of a web page, an application, or a program through a web browser installed in the user system 200. In different embodiments, the data may be provided in such a form as included in a platform in a client-server environment.

The user system 200 is an electronic system providing a user interface for requesting information about the sensitivity of the biological individual to the anticancer drug and displaying data of evaluation results, and it may include at least one of a smart phone, a tablet PC (Personal Computer), a laptop, and/or a PC.

The user system 200 may receive the evaluation results on anticancer drug sensitivity of the biological individual from the system 100 for evaluation of anticancer drug sensitivity and display the received results through a display unit. In this regard, the evaluation results may include the outputs of the sensitivity prediction model such as the degree of sensitivity to anticancer drug A (e.g., high, middle, or low sensitivity; or positive or negative treatment response), and furthermore a sensitivity index to anticancer drug A (e.g., 50 % (-100% ~ 100%)) for determination of the degree of sensitivity to the anticancer drug A.

Hereinafter, a detailed description will be given as to the components of the system 100 for evaluation of anticancer drug sensitivity according to the present invention with reference to FIG. 1b.

Referring to FIG. 1b, the system 100 for evaluation of anticancer drug sensitivity includes a storage unit 110, a communication unit 120, and a processor 130.

The storage unit 110 can store a variety of data produced during the process of determining the degree of sensitivity of the biological individual to an anticancer drug. For example, the storage unit 110 may be configured to store the sensitivity characteristic extracted from the biological test data by the sensitivity prediction model and furthermore the evaluation results on the degree of sensitivity. In different embodiments, the storage unit 110 may include at least one storage medium selected from the group consisting of flash memory, hard disk, MultiMediaCard (MMC) micro, card type memory (e.g., SD or XD memory), RAM, SRAM, ROM, EEPROM, PROM, magnetic memory, magnetic disk, and optical disk.

The communication unit 120 provides a connection to enable communications between the system 100 for evaluation of anticancer drug sensitivity and an external system. The communication unit 120 is connected to the user system 200 and further to the database providing server 300 to receive and transmit different data via wire/wireless communications. More specifically, the communication unit 120 can receive the biological test data of the biological individual and further the reference data of clinical trial results from the database providing server 300. Further, the communication unit 120 can also send evaluation results to the user system 200.

The processor 130 is operably connected to the storage unit 110 and the communication unit 120 to execute different commands for analyzing the biological test data of the biological individual, extracting the related sensitivity characteristic, and determining the degree of sensitivity to the anticancer drug based on the sensitivity characteristic.

More specifically, the processor 130 may be configured to classify the sensitivity characteristic based on the biological test data received from the communication unit 120 and determine the degree of sensitivity to the anticancer drug. For example, the sensitivity characteristic may be a fitting line created by performing a curve fitting of the biological test data including cell growth factors and anticancer response factors of the cell through a regression analysis. In this case, the cell growth factor is a conception that includes both the factor indicating cell proliferation, such as cell growth rate, and the Z score (statistical indicator) of the factor. And, the anticancer drug response factor is a conception that includes both the factor indicating the efficacy of an anticancer drug, such as $IC_{50}$, $\%IC_{50}$, AUC (Area Under the Curve), or variation in cell viability, and the Z score (statistical index) of the factor.

In this regard, the processor 130 may be based on a sensitivity prediction model configured to determine the degree of sensitivity to an anticancer drug based on the biological test data.

On the other hand, the system 100 for evaluation of anticancer drug sensitivity is not limited to an entirely hardware-based system. For example, the processor 130 of the evaluation system 100 may take the form of a software embodiment. Therefore, the evaluation results on the resistance to the anticancer drug may be presented through a display unit of the user system 200, which will be described later.

Referring to FIG. 1c, the user system 200 includes a communication unit 210, a display unit 220, a storage unit 230, and a processor 240.

The communication unit 210 may be configured to enable the user system 200 to communicate with an external system. The communication unit 210 is connected to the system 100 for evaluation of anticancer drug sensitivity via wire/wireless communications to send different data associated with the anticancer drug sensitivity. More specifically, the communication unit 210 may receive from the evaluation system 100 the evaluation results related to the anticancer drug sensitivity of a biological individual, such as the degree of sensitivity (high, middle, or low) of the biological individual to an anticancer drug, or whether the treatment response is positive or negative, and further an evaluation chart for the efficacy of the anticancer drug that presents the degree of sensitivity to the anticancer drug.

The display unit 220 displays a variety of interface images for presentation of evaluation results related to the anticancer drug sensitivity of the biological individual. For example, the display unit 220 may display and provide the degree of sensitivity (high, middle, or low) of the biological individual to an anticancer drug, or whether the treatment response is positive or negative, and further an evaluation chart for the efficacy of the anticancer drug that presents the degree of sensitivity to the anticancer drug.

In different embodiments, the display 220 may include a tough screen that receives touch, gesture, approach, drag, swipe, or hovering inputs using, for example, an electronic pen or a part of the user's body.

The storage unit 230 can store a variety of data used to provide a user interface for presentation of resultant data. In different embodiments, the storage unit 230 may include at least one storage medium selected from the group consisting of flash memory, hard disk, MultiMediaCard (MMC) micro, card type memory (e.g., SD or XD memory), RAM (Random Access Memory), SRAM (Static Random Access Memory), ROM (Read-Only Memory), EEPROM (Electrically Erasable Programmable Read-Only Memory), PROM (Programmable Read-Only Memory), magnetic memory, magnetic disk, and optical disk.

The processor 240 is operably connected to the communication unit 210, the display unit 220, and the storage unit 230 and capable of executing different commands for providing a user interface for presentation of resultant data.

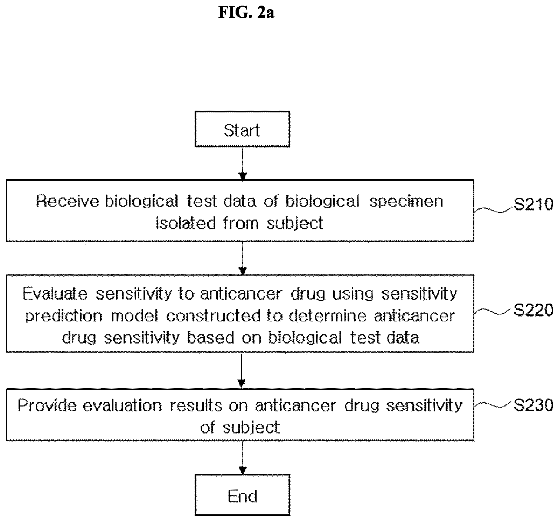

Hereinafter, a detailed description will be given as to a method for evaluation of anticancer drug sensitivity according to an embodiment of the present invention with reference to FIGS . 2a to 2f. FIGS. 2a to 2f are exemplary illustrations showing the procedures of the method for evaluation of anticancer drug sensitivity according to an embodiment of the present invention.

Referring to FIG. 2a, the procedures of the method for evaluation of anticancer drug sensitivity according to an embodiment of the present invention are as follows. Firstly, biological test data of a biological individual are received, in step S210. Then, a sensitivity prediction model is adopted to evaluate the anticancer drug sensitivity of the biological individual based on the biological test data, in step S220. Finally, the evaluation results are provided, in step S230.

More specifically, biological test data such as the name, concentration and dilution ratio of the anticancer drug and the cell viability may be received in the step S210 of receiving biological test data.

According to a feature of the present invention, the biological test data received in the step S210 may include biological test results and test condition data for evaluation on the efficacy of the anticancer drug for a cancer cell isolated from a biological individual with ovarian cancer or breast cancer. In this regard, the anticancer drug may be, if not limited to, doxorubicin.

Preferably, biological test data of a cancer cell may be received in the step S210 of receiving biological test data, where the cancer cell may be cultured on a culture plate that includes a space for containing a cell culture solution, a plurality of pillar portions projecting in the form of pillars with a defined height from the flat bottom surface and designed to have a cell as a culture subject placed on, and a trench groove portion being concavely formed on the flat bottom surface in the other way of the outwardly bulging pillar portions so that the cell culture solution placed in the containing space is kept from lying around all over the bottom but accumulates at one end of the bottom. This disclosure is not to limit the invention.

On the other hand, according to another feature of the present invention, a step of authenticating a user intending to receive the results of evaluation on the anticancer drug sensitivity of the biological individual may be further performed prior to the step S210 of receiving biological test data.

In other words, in the step S210 of receiving biological test data, the biological test data of the biological individual may be received after the authenticated user logging in.

Then, in the step S220 of evaluating sensitivity to the anticancer drug, the anticancer drug sensitivity of the biological individual may be determined in terms of whether treatment response is positive or negative, by using a sensitive prediction model that is configured to receive the anticancer drug response factor and the cell's growth factor as input data and output the degree of sensitivity to an anticancer drug.

According to a feature of the present invention, in the step S220 of evaluating sensitivity to the anticancer drug, a sensitivity characteristic may be extracted based on the biological test data by means of the sensitivity prediction model, and the sensitivity of the biological individual to the anticancer drug may be determined in terms of whether treatment response is positive or negative based on the sensitivity characteristic.

The sensitivity characteristic, for example, may be a fitting line created by performing a curve fitting of the biological test data including cell growth factors and anticancer response factors of the cell. Alternatively, according to another feature of the present invention, the curve fitting in the step S220 of evaluating sensitivity to the anticancer drug may be performed by at least one of LR (Logistic regression), PR (Probit regression), Quadratic classifiers, Kernel estimation, LVQ (Learning vector quantization), ANN (Artificial neural networks), RF (random forest), Bagging (bootstrap aggregating), AdaBoost, Gradient Boosting, XGBoost, SVM (support vector machine), LASSO (least absolute shrinkage and selection operator), Ridge (ridge regression), and Elastic Net. In this case, the degree of anticancer drug sensitivity may depend on the fitting line determined by the curve fitting. In addition, the cell growth factor is a conception that includes both the factor indicating cell proliferation, such as cell growth rate, and the Z score (statistical indicator) of the factor. And, the anticancer drug response factor is a conception that includes both the factor indicating the efficacy of an anticancer drug, such as $IC_{50}$, $\%IC_{50}$, AUC (Area Under the Curve), or variation in cell viability, and the Z score (statistical index) of the factor.

According to further another feature of the present invention, reference data of clinical trial results may be received prior to the step S220 of evaluating anticancer drug sensitivity; and the sensitivity of the biological individual to the anticancer drug may be evaluated based on the biological test data (or sensitivity characteristic) and the reference data according to the sensitivity prediction model in the step S220 of evaluating anticancer drug sensitivity.

According to further another feature of the present invention, the degree of treatment response of the biological individual to the anticancer drug may be determined as being at least one of high, middle and low according to the sensitivity prediction model in the step S220 of evaluating sensitivity to the anticancer drug.

In this regard, the degree of treatment response may be determined based on the sensitivity index to the anticancer drug.

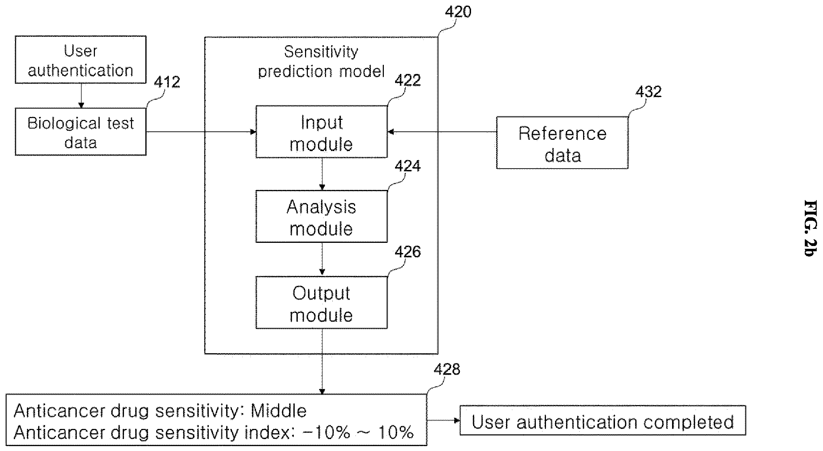

Referring to FIG. 2b, in the step S220 of evaluating sensitivity to the anticancer drug, the received biological test data 412 are fed to a sensitivity prediction model 420 after the user is authenticated by logging in. In this regard, the biological test data 412 may be fed to an input module 422 of the sensitivity prediction model 420. The input module 422, on the other hand, may further receive reference data of clinical trial results 432. Then, an analysis module 424 may extract a sensitivity characteristic from the biological test data 412; and an output module 426 may determine and output the degree of anticancer drug sensitivity based on the sensitivity characteristic. Here, the output module 426 may determine the degree of treatment response of the biological individual to the anticancer drug as being high, middle or low according to the reference data of clinical trial results 432 in addition to the sensitivity characteristic extracted from the biological test data 412 and provide analysis results 428. More specifically, the analysis module 424 calculates the sensitivity index to the anticancer drug from the biological test data 412; and the output module 426 determines the degree of treatment response as being "low" for the sensitivity index ranging from -100% to -10%, "middle" for the sensitivity index from -10% to +10%, and "high" for the sensitivity index from +10% to +100%.

But, the method for evaluation of the degree of treatment response is not limited to the disclosure above. For example, the output module 426 may output whether treatment response is positive or negative based on multiple factors, including the degree of drug response of the cell such as anticancer drug factors (e.g., $IC_{50}$, $\%IC_{50}$, or the rate of change of cell viability) and cell growth factors (e.g., cell growth rate); or determine treatment response in terms of statistics . Further, the output module 426 may determine an evaluation chart for the efficacy of the anticancer drug that presents the degree of sensitivity to the anticancer drug.

According to another feature of the present invention, the degree of treatment response may be determined based on the cell growth factor and the drug response factor in the step S220 of evaluating sensitivity to the anticancer drug.

More specifically, the degree of treatment response may be determined based on the "cell growth factor" consisting of cell growth rate or growth-related markers such as Ki67, and the "anticancer drug response factor" consisting of $IC_{50}$, $\%IC_{50}$ (= ($IC_{50}$ / highest concentration) x 100), cell viability in a specific drug concentration A (= (cell viability at concentration A / cell viability without drug treatment) x 100), or the rate of change of cell viability at a concentration A (= (cell viability / initial cell viability) x 100).

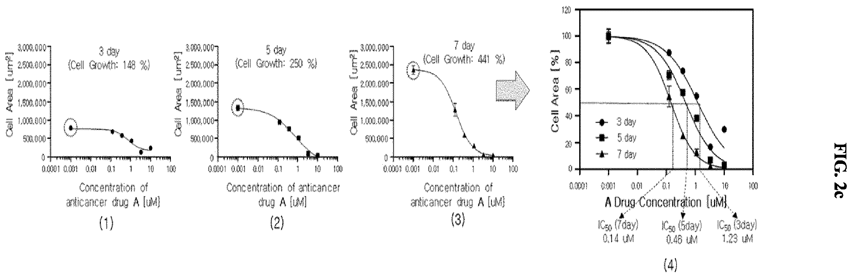

In more detail, referring to (1), (2) and (3) of FIG. 2c that illustrates a representative example of a system for evaluation of anticancer drug sensitivity proposed in the present invention, the measurements of the cell's response to the anticancer drug are varied by date as shown in the graphs obtained by treating a cell line responsive to anticancer drug A with the anticancer drug A by concentration and observing it for 3, 5 and 7 days. It can be understood that the result is because the growth rate by date affects the response to the anticancer drug.

The cell area that indicates the cell viability by date in (1), (2) and (3) of FIG. 2c varies at the minimum concentration of the anticancer drug. Such a cell growth may result in equalizing the cell viability at each concentration based on the cell viability at the concentration of the anticancer drug and varying the response to the anticancer drug, as shown in (4) of FIG. 2c that presents the measurements of the response to the anticancer drug.

Hence, $IC_{50}$ (the concentration at which half of the cells die), one of the anticancer responses in cells, is affected by the cell growth even with the same cell line and the same anticancer drug.

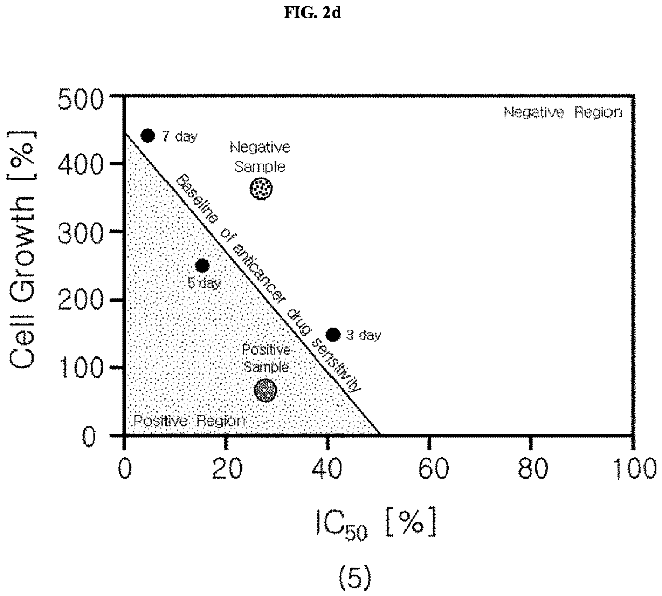

As the response to the anticancer drug changes depending on the cell growth as described above, the growth plotted by taking the cell growth and the anticancer response is shown as FIG. 2d.

Therefore, the model according to an embodiment of the present invention, as shown in FIG. 2d, distinguishes between a positive region (a group sensitive to the anticancer drug) and a negative region (a group resistant to the anticancer drug) of the anticancer response in consideration of the cell growth even with the samples having the same anticancer response ($IC_{50}$ value) by acquiring a sensitivity baseline according to the drug. That is, the anticancer response can be classified into positive or negative as the cell growth varies even with the same anticancer response ($IC_{50}$ value),

as shown in FIG. 2d.

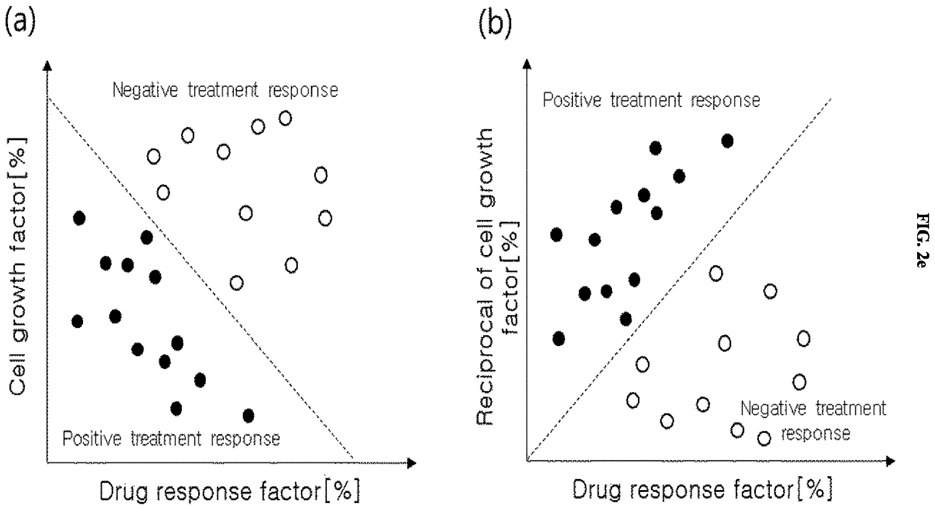

Referring to (a) and (b) of FIG. 2e, the treatment response prediction model may be selected from a model ((a) of FIG. 2e) predicting the presence of a treatment response based on the cell growth factor and the drug response factor and a model ((b) of FIG. 2e) predicting whether the treat response exists based on the reciprocal of the cell growth factor and the drug response factor.

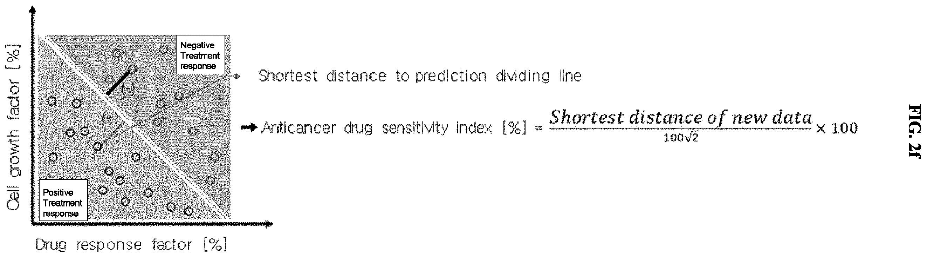

Referring further to FIG. 2f, the prediction model (FIG. 2d and (b) of FIG. 2e) that predicts whether the treat response exists based on the reciprocal of the cell growth factor and the drug response factor may calculate the anticancer drug sensitivity index based on the shortest distance of the new data from the determined prediction dividing line (so-called "fitting line") according to the following Equation 1:

[Equation 1]

$$\text{Anticancer drug sensitivity index (\%)} = \frac{\textit{Shortest distance of new data}}{100\sqrt{2}}$$

The anticancer drug sensitivity index is dependent upon the shortest distance between the position of the biological individual according to the cell growth factor (reciprocal) and the degree of drug response and the prediction dividing line.

In this case, half of $100\sqrt{2}$, which is the distance between 100 on the x-axis and 100 on the y-axis, can be used as the maximum distance in order to normalize the anticancer drug sensitivity index into a percent of the total.

In another example, in normalization, the maximum distance can be the half-distance between the x-intercept and y-intercept of the baseline of anticancer drug sensitivity, or half of the distance between the maximum values on the x-axis and y-axis in the data range.

The system for evaluation of anticancer drug sensitivity according to the present invention may be configured to determine that the biological individual is in an unpredictable region when it is closer to the prediction dividing line (fitting line) at a certain distance or less even though it is evaluated as being in the region of a positive treatment response or a negative treatment response. The biological individual (specimen), if belonging to an obscure region, is excluded from the evaluation, in which case the overall accuracy of prediction on the treatment response may be increased.

The degree of treatment response is evaluated as being "low" for the sensitivity index ranging from -100% to -10%, "middle" for the sensitivity index from -10% to +10%, and "high" for the sensitivity index from +10% to +100%, which disclosure is not to be regarded as limits to the present invention.

According to further another feature of the present invention, in the step S220 of evaluating sensitivity to the anticancer drug, the degree of anticancer drug sensitivity may be determined by a sensitivity prediction model based on at least one algorithm selected from LR (Logistic regression), PR (Probit regression), Quadratic classifiers, Kernel estimation, LVQ (Learning vector quantization), ANN (Artificial neural networks), RF (random forest), Bagging (bootstrap aggregating), AdaBoost, Gradient Boosting, XGBoost, SVM (support vector machine), LASSO (least absolute shrinkage and selection operator), Ridge (ridge regression), and Elastic Net. Preferably, the sensitivity prediction model is, if not limited to, a classification model based on LR algorithm.

Referring back to FIG. 2a, the evaluation results about the anticancer drug sensitivity of the biological individual according to the sensitivity prediction model may be provided in the step S230 of providing evaluation results.

According to a feature of the present invention, the step S230 of providing evaluation results may include providing the degree of sensitivity (high, middle, or low) of the biological individual to an anticancer drug, or whether the treatment response is positive or negative, and further an evaluation chart for the efficacy of the anticancer drug that presents the degree of sensitivity to the anticancer drug.

Referring back to FIG. 2b, for example, the analysis results 428 acquired by the output module 426 of the sensitivity prediction model 420 may be sent to the user system and provided through the display unit of the user system, in the step S230 of providing evaluation results.

According to another feature of the present invention, the user may be logging out after the step S230 of providing evaluation results.

The system for evaluation of anticancer drug sensitivity according to different embodiments as described above makes it possible to predict and provide the degree of anticancer drug sensitivity with high accuracy. For this reason, the present invention provides the system for evaluation of anticancer drug sensitivity and thus overcomes the limitations of the conventional system for evaluation of anticancer drug sensitivity that is based on a single factor such as $IC_{50}$ and presents evaluation results with such a low reliability that there is a mismatch between the evaluation results and the clinical trial results of the biological individual. Further, the system for evaluation of anticancer drug sensitivity according to the present invention enables medical workers to rapidly choose a suitable therapeutic agent according to the evaluation results and thus contributes to early treatments and excellent prognosis.

[Evaluation: Evaluation Results of Systems for Evaluation of Anticancer Drug Sensitivity According to Different Embodiments of Present Invention]

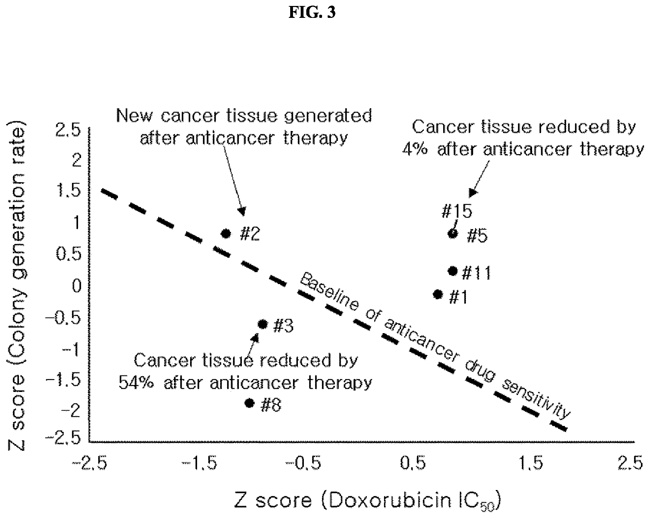

Hereinafter, reference will be made to FIG. 3 to describe the evaluation results of the system for evaluation of anticancer drug sensitivity according to different embodiments of the present invention. FIG. 3 presents the evaluation results of the system for evaluation of anticancer drug sensitivity according to different embodiments of the present invention

Here, the system for evaluation of anticancer drug sensitivity may be based on a sensitivity prediction model that uses an LR (Logistics Regression) algorithm programmed to extract a sensitivity characteristic associated with the anticancer drug response factor and the cell growth factor based on the biological test data such as cancer cell viability in addition to the anticancer drug's name, concentration and dilution ratio and output the degree of sensitivity to the anticancer drug as being high, middle or low based on the sensitivity characteristic. Yet, the learning conditions of the sensitivity prediction model are not limited to this disclosure .

Referring to FIG. 3, there are presented evaluation results about the sensitivity to doxorubicin (DOX) in seven biological individuals diagnosed with breast cancer (#1, #2, #3, #5, #8, #11, #15) based on the system for evaluation of sensitivity according to an embodiment of the present invention using the cell colony growth rate as a cell growth factor and the $IC_{50}$ value as an anticancer sensitivity factor. In this regard, it is desirable to consider that the biological individuals above the baseline of anticancer drug sensitivity had low anticancer drug sensitivity, whereas those below the baseline of anticancer drug sensitivity had high anticancer drug sensitivity.

More specifically, patient tissue #2 was considered to have high doxorubicin sensitivity due to its low $IC_{50}$ value but confirmed to have a new mass after the anticancer therapy according to clinical trial trial results. This implicitly showed that there were limitations in predicting clinical trial results using only anticancer response factors. However, when growth factors were taken into consideration, patient tissue #2 displayed 100% colony generation rate and positioned above the baseline of anticancer sensitivity, so it was considered to have low anticancer drug sensitivity. The measurement results were similar to the clinical trial results.

Furthermore, patient tissues #15, #5, #11, and #1 were above the baseline of anticancer drug sensitivity and considered to have low anticancer drug sensitivity, whereas patient tissues #3 and #8 were below the baseline of anticancer drug sensitivity and considered to have high anticancer drug sensitivity.

While the present invention has been particularly illustrated and described with reference to exemplary embodiments thereof, various modifications or changes can be made without departing from the scope of the present invention. The disclosed embodiments of the present invention are given in order to explain the technical conception of the present invention rather than to limit the conception of the invention. Therefore, the present invention is not confined to the disclosed embodiments and should be construed as including all the technical conceptions included in the scope of the present invention.

100: Evaluation system for anticancer drug sensitivity
110, 230: Storage unit
120, 210: Communication unit
130, 240: Processor
200: User system
220: Display unit
330: Database providing server
412: Biological test data
420: Sensitivity prediction model
422: Input module
424: Analysis module
426: Output module
428: Analysis results
432: Reference data
1000: Evaluation system for treatment response to anticancer drug

**Claims**

1.  A system for evaluating anticancer drug sensitivity, comprising:

a communication unit configured to receive biological test data according to cell experiments for a biological specimen isolated from a biological individual; and

a processor connected to the communication unit,

the processor being configured to determine sensitivity to an anticancer drug in the biological individual in terms of whether treatment response is positive or negative based on the biological test data by using a sensitivity prediction model configured to determine sensitivity to an anticancer drug based on an anticancer drug response factor and a cell growth factor according to cell experiments, and then to provide evaluation results on the sensitivity to the anticancer drug in the biological individual.

2. The system according to claim 1, wherein the biological test data according to cell experiments are at least one selected from the group consisting of the name of the anticancer drug, the concentration of the anticancer drug, the dilution ratio of the anticancer drug, and cell viability.

3. The system according to claim 1, wherein the sensitivity prediction model is further configured to extract a sensitivity characteristic associated with the anticancer drug response factor and the cell growth factor based on the biological test data,

the anticancer drug response factor being at least one of $IC_{50}$, $\%IC_{50}$, cell viability rate, and the rate of change of cell viability,

the cell growth factor being at least one selected from the rate of increase in cell viability, the cell growth rate, the rate of increase in cell size, or the colony generation rate that indicate the cell growth according to cell experiments,

wherein the processor is further configured to use the sensitivity prediction model to extract the sensitivity characteristic based on the biological test data and determine the anticancer drug sensitivity of the biological individual in terms of whether treatment response is positive or negative.

4. The system according to claim 3, wherein the sensitivity characteristic is a fitting line created by performing a curve fitting for the biological test data including the cell growth factor and the anticancer drug response factor of the cell.

5. The system according to claim 4, wherein the curve fitting is performed by at least one selected from the group consisting of LR (Logistic regression), PR (Probit regression), Quadratic classifiers, Kernel estimation, LVQ (Learning vector quantization), ANN (Artificial neural networks), RF (random forest), Bagging (bootstrap aggregating), Ada-Boost, Gradient Boosting, XGBoost, SVM (support vector machine), LASSO (least absolute shrinkage and selection operator), Ridge (ridge regression), and Elastic Net.

6. The system according to claim 1, further comprising a receiver for receiving reference data of clinical trial results for the biological individual,

wherein the processor is further configured to use the sensitivity prediction model to determine the anticancer drug sensitivity of the biological individual in terms of whether treatment response is positive or negative based on the biological test data and the reference data.

7. The system according to claim 1, wherein the processor is further configured to use the sensitivity prediction model to determine the degree of treatment response to the anticancer drug as being at least one of high, middle and low and then to provide the degree of treatment response to the anticancer drug determined by the sensitivity prediction model.

8. The system according to claim 1, wherein the anticancer drug is a drug used for anticancer therapy, including doxorubicin,

the biological individual being an entity with cancer, including ovarian cancer, lung cancer, stomach cancer, or breast cancer.

9. A computer-readable medium storing a program executing a method for evaluation of anticancer drug sensitivity, in which the evaluation method for anticancer drug sensitivity is implemented by a receiver and a processor, the evaluation method comprising:

(a) through the receiver, receiving biological test data according to cell experiments of a biological specimen isolated from a biological individual;

(b) through the processor, determining anticancer drug sensitivity of the biological individual in terms of whether treatment response is positive or negative based on the biological test data according to cell experiments, by using a sensitivity prediction model configured to determine sensitivity to an anticancer drug based on an anticancer drug response factor and a cell growth factor; and
(c) providing results of evaluation on the anticancer drug sensitivity of the biological individual.

10. The computer-readable medium according to claim 9, wherein the biological test data according to cell experiments are at least one selected from the group consisting of the name of the anticancer drug, the concentration of the anticancer drug, the dilution ratio of the anticancer drug, and cell viability.

11. The computer-readable medium according to claim 9, wherein the sensitivity prediction model is further configured to extract a sensitivity characteristic associated with the anticancer drug response factor and the cell growth factor based on the biological test data,

the anticancer drug response factor being at least one of $IC_{50}$, $\%IC_{50}$, cell viability rate, and the rate of change of cell viability,

the cell growth factor being at least one selected from the rate of increase in cell viability, the cell growth rate, the rate of increase in cell size, or the colony generation rate that indicate the cell growth according to cell experiments,

wherein the step of determining the anticancer drug sensitivity in terms of whether treatment response is positive or negative further comprises:

using the sensitivity prediction model to extract the sensitivity characteristic based on the biological test data; and
determining the anticancer drug sensitivity of the biological individual in terms of whether treatment response is positive or negative based on the sensitivity characteristic.

12. The computer-readable medium according to claim 11, wherein the sensitivity characteristic is a fitting line created by performing a curve fitting for the biological test data including the cell growth factor and the anticancer drug response factor of the cell.

13. The computer-readable medium according to claim 12, wherein the curve fitting is performed by at least one selected from the group consisting of LR (Logistic regression), PR (Probit regression), Quadratic classifiers, Kernel estimation, LVQ (Learning vector quantization), ANN (Artificial neural networks), RF (random forest), Bagging (bootstrap aggregating), AdaBoost, Gradient Boosting, XGBoost, SVM (support vector machine), LASSO (least absolute shrinkage and selection operator), Ridge (ridge regression), and Elastic Net.

14. The computer-readable medium according to claim 9, wherein the evaluation method further comprises additionally receiving reference data of clinical trial results for the biological individual prior to determining the anticancer drug sensitivity in terms of whether treatment response is positive or negative,

wherein the step of determining the anticancer drug sensitivity in terms of whether treatment response is positive or negative further comprises using the sensitivity prediction model to determine the anticancer drug sensitivity of the biological individual in terms of whether treatment response is positive or negative based on the biological test data and the reference data.

15. The computer-readable medium according to claim 9, wherein the step of determining the anticancer drug sensitivity in terms of whether treatment response is positive or negative further comprises using the sensitivity prediction model to determine the degree of treatment response to the anticancer drug as being at least one of high, middle and low,

wherein the step of providing the results of evaluation on the anticancer drug sensitivity comprises providing the degree of treatment response to the anticancer drug determined by the sensitivity prediction model.

16. The computer-readable medium according to claim 9, wherein the anticancer drug is doxorubicin, the biological individual being an entity with ovarian cancer or breast cancer.

**FIG. 1a**

1000

Name: Hong, xx
Age: 57 years old
Predicted treatment response to anticancer drug A: High
(positive treatment response)
Sensitivity index to anticancer drug A: 50 % (-100% ~
100%)

200

100

300

**FIG. 1b**

100

Storage unit — 110

Processor — 130

Communication unit — 120

**FIG. 1c**

**FIG. 2a**

```
                    ┌──────────────┐
                    │    Start     │
                    └──────┬───────┘
                           │
                           ▼
┌─────────────────────────────────────────────────┐
│  Receive biological test data of biological  specimen │   S210
│            isolated  from  subject            │
└─────────────────────────┬───────────────────────┘
                           │
                           ▼
┌─────────────────────────────────────────────────┐
│  Evaluate  sensitivity to anticancer  drug using  sensitivity │   S220
│  prediction  model constructed to determine anticancer │
│       drug  sensitivity based  on biological test data │
└─────────────────────────┬───────────────────────┘
                           │
                           ▼
┌─────────────────────────────────────────────────┐
│  Provide evaluation results on anticancer drug sensitivity │   S230
│                  of  subject                  │
└─────────────────────────┬───────────────────────┘
                           │
                           ▼
                    ┌──────────────┐
                    │     End      │
                    └──────────────┘
```

**FIG. 2b**

**FIG. 2c**

**FIG. 2d**

(5)

**FIG. 2e**

**FIG. 2f**

Shortest distance to prediction dividing line

$$\text{Anticancer drug sensitivity index [\%]} = \frac{\textit{Shortest distance of new data}}{100\sqrt{2}} \times 100$$

Negative Treatment response

Positive Treatment response

(-)

(+)

Cell growth factor [%]

Drug response factor [%]

**FIG. 3**

Europäisches
Patentamt

European
Patent Office

Office européen
des brevets

# EUROPEAN SEARCH REPORT

Application Number

EP 21 17 0607

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BRIDGES ET AL: "The hollow fiber assay for drug responsiveness in the Ewing's sarcoma family of tumors", JOURNAL OF PEDIATRICS, MOSBY-YEAR BOOK, ST. LOUIS, MO, US, vol. 149, no. 1, 1 July 2006 (2006-07-01), pages 103-111, XP005581272, ISSN: 0022-3476, DOI: 10.1016/J.JPEDS.2006.02.042 | 1-4, 6-12, 14-16 | INV.<br>G16H20/10<br>G16H50/20<br>G01N33/574<br>G16C20/30 |
| Y | * abstract * * p.104 col.1 par.3 and section "Cell Culture"; p.105 col.2 section "Evaluation of ESFT HFA Using Doxorubicin"and col.2 par.2; p.106 col.2 par.2; p.108 par. across col.1-2; figures 2-3 * | 5,13 | |
| X | WO 2019/035766 A1 (AGENCY SCIENCE TECH & RES [SG]) 21 February 2019 (2019-02-21) * [004], [0011], [0020], [0076], [0093]-[00100]; figures 7, 11, 19, 20 * * Table 1 on p.22 * * [0034], [0052]; figure 21 * * [00103]-[00108] * | 1-16 | |
| Y | DE NIZ C ET AL: "Algorithms for Drug Sensitivity Prediction", ALGORITHMS, vol. 9, no. 4, 17 November 2016 (2016-11-17), pages 1-25, XP055614617, DOI: 10.3390/a9040077 * abstract * * page 4 * * pages 14-15 * | 5,13 | TECHNICAL FIELDS SEARCHED (IPC)<br><br>G16H<br>G16C<br>G01N |

The present search report has been drawn up for all claims

| Place of search | Date of completion of the search | Examiner |
|---|---|---|
| Munich | 15 October 2021 | Werner, Andreas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 17 0607

This annex lists the patent family members relating to the patent documents cited in the above-mentioned European search report.
The members are as contained in the European Patent Office EDP file on
The European Patent Office is in no way liable for these particulars which are merely given for the purpose of information.

15-10-2021

| Patent document cited in search report | Publication date | Patent family member(s) | Publication date |
|---|---|---|---|
| WO 2019035766 A1 | 21-02-2019 | CN 111194407 A<br>SG 11202001087R A<br>US 2020218874 A1<br>WO 2019035766 A1 | 22-05-2020<br>30-03-2020<br>09-07-2020<br>21-02-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 1020200055230 A